# EUROPÄISCHE PATENTSCHRIFT

(11) **EP 0 562 370 B1**
(45) Veröffentlichungstag und Bekanntmachung des Hinweises auf die Patenterteilung: **26.11.1997**
(21) Anmeldenummer: 93103885.5
(22) Anmeldetag: 10.03.1993
(51) Int. Cl.: G01N 27/327, C12M 1/40, C12Q 1/00

(54) **Biosensor**
Biosensor
Biocapteur

(30) Priorität: 23.03.1992 DE 4209370
(43) Veröffentlichungstag der Anmeldung: 29.09.1993
(73) Patentinhaber: SIEMENS AKTIENGESELLSCHAFT, 80333 München (DE)
(72) Erfinder: von Gentzkow, Wolfgang, Dr. Dipl.-Chem., W-8524 Kleinsendelbach (DE); Feucht, Hans-Dieter, Dr. Dipl.-Chem., W-7253 Renningen (DE)

(56) Entgegenhaltungen:
- EP-A- 0 225 094
- EP-A- 0 291 130
- FR-A- 2 476 125

## Beschreibung

Die Erfindung betrifft einen Biosensor mit einem selektiven Erkennungssystem aus einem Polymer und einer biochemischen Substanz, insbesondere ein Enzym.

Biosensoren sind Chemosensoren mit einem biologischen Erkennungssystem. Dieses Erkennungssystem besteht aus biologisch aktiven Substanzen, wie Enzymen, Antikörpern, Lectinen, Hormonrezeptoren usw., die auf der Oberfläche des Sensors oder in einer darauf befindlichen dünnen Schicht immobilisiert sind. Beim Erkennungsprozeß wird durch Wechselwirkung mit dem zu charakterisierenden gasförmigen oder flüssigen Medium auf der Oberfläche bzw. in dieser Schicht des Sensors eine Veränderung erzeugt, die durch elektrische, optische, mechanische, akustische oder kalorimetrische Meßverfahren beurteilt werden kann. Bei Geräten mit elektronischer Datenerfassung und -auswertung befindet sich zu diesem Zweck die aktive Oberfläche bzw. Schicht als Signalgeber in direkter räumlicher Kopplung mit einem Signalwandler, Transducer genannt, der mit der Auswerteelektronik verbunden ist.

Von der Zuordenbarkeit und Reproduzierbarkeit der in der sensitiven Schicht des Biosensors erzeugten Signale hängt die Zuverlässigkeit des gesamten Sensors ab. Dies bedeutet, daß die Schicht - neben hoher Selektivität und Sensitivität - insbesondere auch eine hysterese- und driftfreie Funktion, chemische und biologische Stabilität sowie Kontaminationsbeständigkeit aufweisen muß. Insbesondere für den technischen Einsatz wird darüber hinaus Bedienungskomfort, Integrierbarkeit und ein möglichst geringer Meß/Regenerations-Zeitbedarf bei hoher Langzeitstabilität gefordert, wobei die Herstellung der Schicht - nach fertigungstechnisch rationellen und automatisierbaren Verfahren - möglichst einfach, reproduzierbar und kostengünstig erfolgen und in den Fertigungsprozeß zur Sensorherstellung integrierbar sein soll.

Bislang haben nur solche Biosensoren eine praktische Bedeutung erlangt, welchen enzymatische Reaktionen zugrunde liegen. Bei diesen Reaktionen wird der Umstand genutzt, daß einfach detektierbare Produkte, wie H⁺, O₂, H₂O₂, CO₂ und NH₃, entstehen oder verbraucht werden. Hinsichtlich Selektivität und Sensitivität entsprechen die enzymatischen Reaktionen voll den Anforderungen. Eine Schwierigkeit besteht aber darin, die Enzyme - ohne Aktivitätsverluste - in einer möglichst dünnen Erkennungsschicht so zu immobilisieren, daß sie für die zu detektierenden Stoffe schnell zugänglich und gegen Vergiftung sowie biochemische Schadstoffe resistent sind und möglichst lange funktionsstabil bleiben.

Zur Immobilisierung von Enzymen sind bislang folgende Methoden beschrieben worden:
- Adsorption an Trägeroberflächen
- ionische Bindung an Trägeroberflächen
- kovalente Bindung an Trägeroberflächen
- Absorption in Polymerschichten
- Einschluß in einem Polymernetzwerk (Matrixeinhüllung, Mikroverkapselung)
- Einschluß durch Umhüllung mit einer Membran (Makroverkapselung)
- Quervernetzung bzw. Copolymerisation mit di- oder polyfunktionellen Monomeren.

Wie aus der umfangreichen Literatur über die Immobilisierung von Enzymen hervorgeht, haben aber alle diese Methoden Nachteile, die sie für eine industrielle Sensorfertigung wenig attraktiv erscheinen lassen (siehe dazu beispielsweise: W. Hartmeier, "Immobilisierte Biokatalysatoren", Springer-Verlag Berlin, Heidelberg 1986, Seiten 23 bis 51, sowie J. Woodward, "Immobilised cells and enzymes", IRL Press, Oxford, Washington DC, 1985, Seiten 3 bis 54).

So ergeben die Adsorption und die ionische Bindung von Enzymen an Oberflächen relativ instabile Systeme von begrenzter Einsatzbreite: Änderungen des pH-Wertes und der Ionenstärke von damit in Kontakt befindlichen Lösungen oder die Anwesenheit anderer Stoffe führen bereits zur Verdrängung des oberflächlich gebundenen Enzyms und damit zu Aktivitätsverlusten des Erkennungssystems. Auch bei der Absorption in Polymerschichten, in der überwiegenden Zahl der Fälle wird dabei weichgemachtes Polyvinylchlorid verwendet (siehe beispielsweise: "Sensors and Actuators", Vol. 18 (1989), Seiten 329 bis 336, und "Ber. Bunsenges. Phys. Chem.", Bd. 92 (1988), Seiten 1423 bis 1426), werden relativ instabile Systeme erhalten: Migration und Extraktion der Enzyme führen zu einer ständigen Aktivitätsabnahme (Drift) und begrenzen die Lebensdauer des Sensors.

Wesentlich stabilere Systeme werden erhalten, wenn die Enzyme kovalent an eine Trägeroberfläche gebunden, über Quervernetzung bzw. Copolymerisation unlöslich gemacht oder durch Mikro- oder Makroverkapselung immobilisiert werden. Für die Bildung kovalenter Bindungen und die Quervernetzung stehen seitens der Enzyme freie Amino-, Carboxyl-, Hydroxyl- und Mercaptogruppen zur Verfügung. Als Trägermaterial können sowohl anorganische Materialien, wie Glas, als auch natürliche und synthetische organische Polymere verwendet werden. Voraussetzung ist dabei, daß die Trägermaterialien reaktive Gruppen, wie Isocyanat-, Isothiocyanat-, Säurechlorid- und Epoxidgruppen, enthalten. Weniger reaktive Gruppen lassen sich aktivieren, beispielsweise Carboxylgruppen durch das Carbodiimid- oder Azidverfahren, Hydroxylgruppen durch die Bromcyanmethode und Aminogruppen durch das Isothiocyanat- oder Azoverfahren. Vor allem auf der Basis von Acryl- und Methacrylsäurederivaten gelang es, zahlreiche reaktive Copolymerisate mit Dinitrofluorphenyl-, Isothiocyanat-, Oxiran- oder Säureanhydridgruppen herzustellen. Kommerziell erhältlich sind beispielsweise Polyacrylamide mit Oxirangruppen sowie modifizierte Copolymerisate auf der Basis von Vinylacetat und Divinylethylenharnstoff mit Oxirangruppen.

Die Immobilisierung durch Quervernetzung bzw. durch Copolymerisation stellen Sonderformen der kovalenten Bindung dar. Bei diesen Methoden erfolgt die Ausbildung kovalenter Bindungen zwischen den Enzymmolekülen und di- oder polyfunktionellen Monomeren, wie Glutardialdehyd, bei der Copolymerisation zusätzlich zwischen den Enzymmolekülen und einer polymerisierenden Substanz. Auf diese Weise entstehen hochmolekulare unlösliche Aggregate. Die Quervernetzung wird als Immobilisierungsmethode im allgemeinen in Kombination mit einer der anderen Methoden angewendet, beispielsweise in Kombination mit der Adsorption oder der Absorption. Hierbei werden die Enzymmoleküle zunächst an der Oberfläche des Trägers adsorbiert oder in einer darauf befindlichen Schicht absorbiert und anschließend miteinander vernetzt.

Ein wesentlicher Nachteil der Immobilisierung durch kovalente Bindung ist die damit verbundene starke Belastung der Biokatalysatoren. Die erforderlichen, zum Teil rauhen Immobilisierungsprozeduren, bei denen ein starker Wechsel des pH-Wertes auftritt, organische Lösungsmittel verwendet werden müssen oder Umsetzungen mit niedermolekularen reaktiven Substanzen erfolgen, führen nämlich fast immer zu starken Konformationsänderungen und damit zu Aktivitätsverlusten derart gebundener Enzyme.

Bei der Immobilisierung durch Einschluß, d.h. Mikro- oder Makroverkapselung, werden nicht die Enzyme selbst unlöslich gemacht, sondern ihr Reaktionsbereich wird durch semipermeable Polymere bzw. Polymerschichten begrenzt. Voraussetzung für die Funktionstüchtigkeit der in dieser Weise eingehüllten Enzyme ist, daß Substrate und Produkte die Hüllsubstanz passieren können, während die Enzyme selbst zurückgehalten werden müssen. Für die Matrixeinhüllung werden neben natürlichen Polymeren, wie Alginat, Carrageenan, Pektin, Agar und Gelatine, die allerdings für eine dauerhafte Immobilisierung von Enzymen zu grobmaschig sind, insbesondere synthetische Polymere, wie Polyacrylamid, verwendet. Zur Verkapselung dienen beispielsweise Polyamide, Polyurethane, Polyester und Polyharnstoffe. Die Einschlußmethode hat den Nachteil, daß relativ dicke Schichten mit langen Sensoransprechzeiten entstehen.

Bei den beschriebenen Methoden wird die Immobilisierung der Enzyme in den meisten Fällen von Hand durchgeführt, was relativ langsam, teuer und wenig reproduzierbar ist und der Integration in moderne Fertigungsprozesse entgegensteht. Hinsichtlich der Vorteile, die Enzymsensoren auf FET-Basis (ENFET's) bieten könnten, sind deshalb in den letzten Jahren Versuche unternommen worden, die Enzymimmobilisierung in die Planartechnik bei der Fertigung von integrierten Schaltungen einzubauen. So ist beispielsweise die Herstellung und direkte Photostrukturierung enzymhaltiger photovernetzbarer Schichten auf Polyvinylalkoholbasis beschrieben ("Proc. 3rd Int. Conf. Solid State Sensors and Actuators (Transducers '85)", June 11-14, 1985, Seiten 148 bis 151). Für den genannten Zweck ist es auch bekannt, photosensitives Polyvinylpyrrolidon zu verwenden ("IEEE Trans. Electron Devices", Vol. ED-36 (1989), Seiten 1303 bis 1310). Nach dieser Methode können auf Wafern Strukturen erzeugt werden, die exakt die Gates der FET's abdecken. Die Methode hat allerdings den großen Nachteil, daß die Enzyme bei der UV-Bestrahlung zumindest partiell inaktiviert werden.

Es ist ferner bekannt, die Enzyminaktivierung durch UV-Strahlung zu nutzen, indem zuerst eine enzymhaltige Schicht aus Acetylcellulose hergestellt, in dieser Schicht das Enzym mit Glutardialdehyd quervernetzt und danach durch eine Maske so bestrahlt wird, daß die Gateabdeckungen abgeschattet werden und damit aktiv bleiben, während die restlichen Flächen inaktiviert werden ("Chemical Economy & Engineering Review", Vol. 17 (1985), No. 7-8, Seiten 22 bis 27). Die inaktivierte Schicht verbleibt dabei auf dem Sensor, was sich als Nachteil für die weitere für den Einsatz erforderliche Isolierung und Verpackung des Sensors erweist.

Beschrieben ist auch die Lift-off-Technik ("Sensors and Actuators", Vol. 13 (1988), Seiten 165 bis 172). Bei dieser Methode wird ein Photoresist so strukturiert, daß nur die Gate-Oberflächen frei bleiben. Darauf wird dann das Enzym zusammen mit Glutardialdehyd aufgebracht und quervernetzt; der Photolack wird mit Hilfe der Lift-off-Technik mit Aceton und Ultraschall entfernt. Dabei läßt sich ebenfalls eine zumindest teilweise Denaturierung des Enzyms nicht vermeiden.

Aufgabe der Erfindung ist es, einen Biosensor mit einem selektiven Erkennungssystem (aus einem Polymer und einer biochemischen Substanz) anzugeben, das technisch einfach, rationell und kostengünstig erzeugt werden kann, wobei das Herstellungsverfahren in moderne Fertigungsverfahren integrierbar sein soll und in reproduzierbarer Weise funktionsstabile, gegebenenfalls auch miniaturisierte und integrierte Erkennungssysteme gleichbleibender Qualität und hoher Lebensdauer liefert.

Dies wird erfindungsgemäß dadurch erreicht, daß ein olefinisch ungesättigter, epoxyfunktioneller Polyether in Form einer Schicht auf ein Trägermaterial aufgebracht wird, daß der Polyether durch energiereiche Strahlung zu einer weitmaschigen epoxyfunktionellen Polymermatrix vernetzt wird, daß die Schicht mit einer wäßrigen Lösung der biochemischen Substanz behandelt wird, wobei die biochemische Substanz durch Reaktion mit Epoxidgruppen in der Polymermatrix immobilisiert wird, und daß die Schicht durch Reaktion von nicht umgesetzten Epoxidgruppen mit einer amino- und/oder carboxylgruppenhaltigen Verbindung stabilisiert wird.

Die Problemlösung besteht bei der Erfindung somit in einer neuen Art der Immobilisierung von Enzymen und anderen biochemischen Substanzen mit selektiven Erkennungseigenschaften, und zwar in Schichten aus strahlenvernetzten epoxyfunktionellen Polyethern. Es wurde nämlich überraschenderweise gefunden, daß diese Substanzen - aus wäßriger Lösung - in weitmaschig vernetzte epoxyfunktionelle Polyether einzudringen vermögen und durch Reaktion mit kettenständigen Epoxidgruppen unter sehr milden Bedingungen in der Polymermatrix, d.h. im Polymernetzwerk, verankert werden können. Diese Tatsache ist völlig neu, und sie eröffnet die Möglichkeit, die Herstellung, Strukturierung und Vernetzung der Schichten vor der Immobilisierung der biochemischen Substanzen durchzuführen und damit eine Schädigung der meistens sehr empfindlichen Substanzen durch die genannten Prozesse zu vermeiden.

Bevorzugte Ausführungsformen des Biosensors nach der Erfindung sind Gegenstand von Unteransprüchen.

Die Erzeugung des Erkennungssystems des erfindungsgemäßen Biosensors beinhaltet im allgemeinen folgende Schritte:
1. Schichtpräparation
   Ein strahlenvernetzbarer, epoxyfunktioneller Polyether oder eine Mischung derartiger Polyether wird, gegebenenfalls in Kombination mit einem Vernetzungsinitiator, einem Vernetzungsverstärker und/oder weiteren Zusatzstoffen, in der gewünschten Schichtdicke auf ein Trägermaterial aufgebracht. Je nach Anwendungsfall und Trägermaterial kann dies aus einer Lösung oder lösungsmittelfrei durch Tauchen, Spin-Coating, Roller-Coating, Curtain-Coating oder einen anderen in der Technik üblichen Prozeß erfolgen, wobei die Vorbehandlung der Trägeroberfläche mit einem Haftvermittler erforderlich sein kann. Die Schichtdicke kann über die Einstellung der Viskosität und durch Zusatz eines Lösemittels oder eines Reaktivverdünners gesteuert werden. Die auf diese Weise hergestellte Schicht muß in jedem Fall von flüchtigen Bestandteilen befreit werden, was beispielsweise durch Trocknen oder Entgasen erfolgen kann.
2. Vernetzung der Schicht
   Die Vernetzung der Schicht, d.h. des Polyethers, erfolgt durch energiereiche Strahlung, insbesondere durch UV-, Elektronen- und γ-Strahlen. Dabei werden ausschließlich die olefinisch ungesättigten, radikalisch polymerisierbaren Gruppen umgesetzt, während die Epoxidgruppen quantitativ erhalten bleiben. Durch die Vernetzung entsteht ein weitmaschiges Polymernetzwerk. Bei Projektionsbelichtung oder Bestrahlung durch eine Maske und nachfolgendes Herauslösen der unvernetzten Bereiche kann die Schicht auch strukturiert werden.
3. Immobilisierung der biochemischen Substanz
   Beim Kontaktieren der vernetzten Schicht mit einer wäßrigen Lösung der biochemischen Substanz wandert diese Substanz in die Polymermatrix hinein und wird dort durch Reaktion mit den Epoxidgruppen kovalent gebunden. Voraussetzung für diesen Prozeß ist - neben der erforderlichen Maschenweite - eine ausreichende Hydrophilie des bei der Vernetzung gebildeten Polymernetzwerkes. Die Immobilisierung kann deshalb durch eine vorangehende Hydrophilierung des Polyethers beschleunigt werden. Dies erfolgt durch Umsetzung eines Teils der Epoxidgruppen mit hydrophilen Verbindungen, die reaktive Gruppen, wie NH-, OH-, SH- oder COOH-Gruppen, enthalten, womit der hydrophile Charakter der Polymerschicht erhöht wird. Der Immobilisierungsprozeß kann auch durch Zusatzstoffe, wie Polyvinylpyrrolidon, die eine erhöhte Wasseraufnahme der Polyether bewirken, sowie durch mit Wasser mischbare Lösemittel, wie Dioxan, Tetrahydrofuran, Alkohole oder Polyether, erheblich beschleunigt werden. Im übrigen können in einer Schicht auch mehrere unterschiedliche biochemische Substanzen immobilisiert werden, was gleichzeitig oder nacheinander erfolgen kann.
4. Stabilisierung der Schicht
   Dieser Schritt beinhaltet die Reaktion von nach der Immobilisierung verbliebenen Epoxidgruppen mit einer amino- und/ oder carboxylgruppenhaltigen Verbindung, insbesondere einer Aminosäure. Die Stabilisierung kann - in Abhängigkeit von der verwendeten Verbindung - zu einer engeren Vernetzung der Schicht und damit zu einer verbesserten mechanischen Festigkeit oder zur Anpassung der Materialeigenschaften und des Stofftransports genutzt werden. Im übrigen ist eine oberflächliche Abdeckung der Sensorschicht durch eine oder mehrere zusätzliche Schichten möglich, was auch zur Einstellung definierter Diffusionsbedingungen sinnvoll sein kann.

Für den erfindungsgemäßen Biosensor eignen sich insbesondere epoxyfunktionelle Polyether folgender Struktur, die in der gleichzeitig eingereichten europäischen Patentanmeldung EP-A-0 562 368 - beschrieben sind:

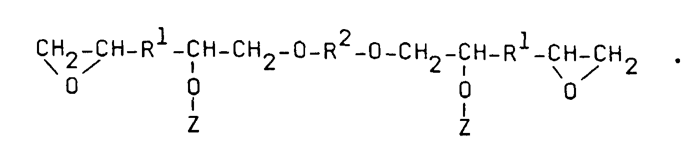

Dabei gilt folgendes:
- Z =: 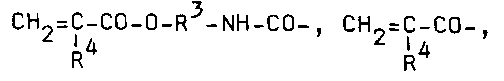 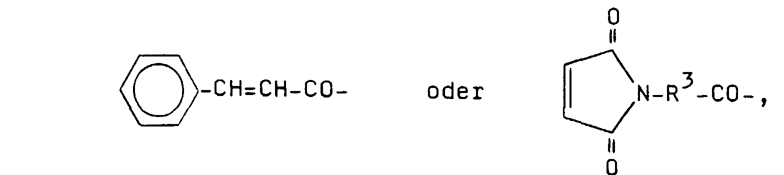 wobei
R³ = -(CH₂)ₘ-, mit m = 1 bis 10,
R⁴ = H oder CH₃;
- R¹ =: -(CH₂)ₒ-, mit o = 0 bis 18,
-CH₂-O-R⁵-O-CH₂-, wobei
R⁵ = -(CH₂)ₚ-, 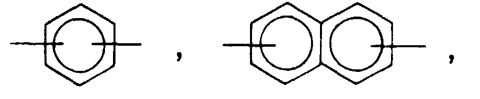 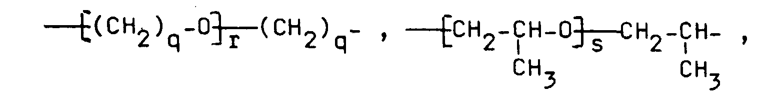 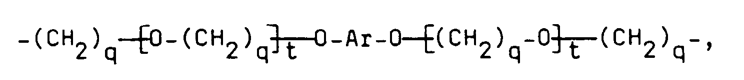 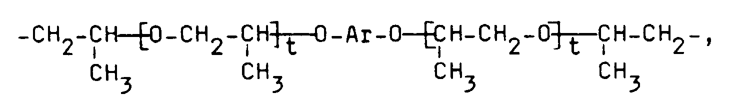 mit
p = 2 bis 20, q = 2 bis 4, r = 1 bis 50,
s = 0 bis 50, t = 0 bis 25, 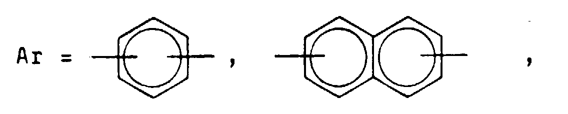 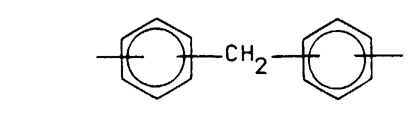 oder 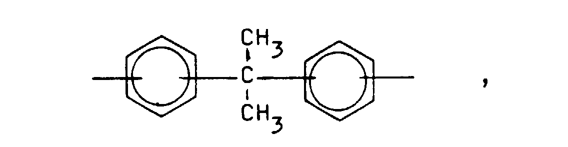 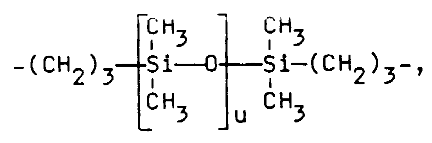 mit u = 0 bis 150,
oder die entsprechende Gruppierung aus 3,4-Epoxycyclohexylmethyl-3',4'-epoxycyclohexancarboxylat, d.h. der Verbindung: 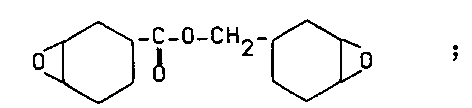
- R² =: -(CH₂-CH=CH-CH₂)ₙ- , -R⁶- , -R⁶-O-CO-R⁷-CO-O-R⁶- oder 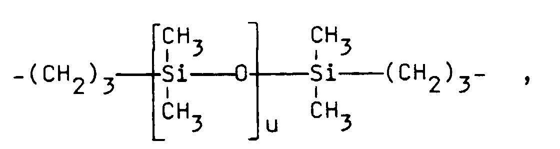 wobei
n = 1 bis 50, u = 0 bis 150,
R⁶ dieselbe Bedeutung hat wie R⁵, außer 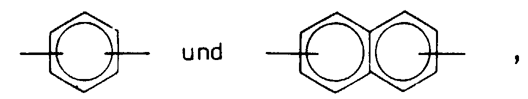 und
R⁷ folgendes bedeutet: 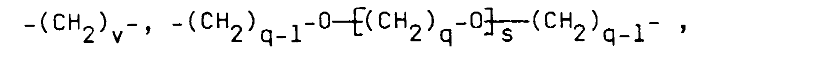 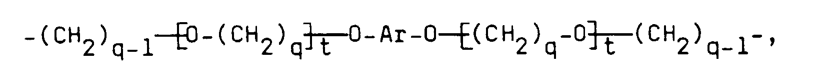 mit
q = 2 bis 4, s = 0 bis 50, t = 0 bis 25,
v = 0 bis 20, und
Ar die vorstehend angegebene Bedeutung hat.

Die Erfindung bietet folgende Vorteile:
- Es wird die Immobilisierung aller biochemischen Substanzen, die an der Peripherie über reaktive NH-, OH-, SH- oder COOH-Gruppen verfügen, ermöglicht.
- Die die immobilisierten biochemischen Substanzen aufweisenden Schichten können auch trocken und unter nicht-sterilen Bedingungen ohne Schädigung dieser Substanzen aufbewahrt werden.
- Die Immobilisierung der biochemischen Substanzen erfolgt unter sehr milden Bedingungen in wäßriger Lösung und in Abwesenheit niedermolekularer reaktiver Komponenten; dadurch werden Verluste, beispielsweise durch Enzymdenaturierung, vermieden.
- Für die Immobilisierung einer großen Zahl verschiedenartiger biochemischer Substanzen und für verschiedene Sensortypen dient eine relativ kleine Zahl großtechnisch herstellbarer und damit kostengünstig zugänglicher Polymermaterialien hoher chemischer und thermischer Stabilität.
- Die Herstellung und Vernetzung sowie gegebenenfalls Strukturierung der Schichten kann nach der Planartechnik, d.h. technisch einfach, reproduzierbar und kostengünstig sowie in die Sensorfertigung integrierbar durchgeführt werden.
- Die Immobilisierung der biochemischen Substanzen kann - je nach Bedarf und Einsatzzweck - unabhängig von der Schichtherstellung erfolgen, gegebenenfalls auch erst beim Anwender, kurz vor dem Einsatz.
- Durch die chemische Verankerung der biochemischen Substanzen in der Polymermatrix werden Desorptions-, Migrations- und Extraktionsverluste vermieden.
- Durch die Ausbildung kovalenter Bindungen zwischen den peripheren NH-, OH-, SH- und COOH-Gruppen der biochemischen Substanzen und dem sehr weichen und flexiblen umhüllenden Polymermaterial wird den zum Teil sehr empfindlichen Substanzen, beispielsweise Enzyme, eine hohe Funktions- und Langzeitstabilität verliehen.
- Durch die Möglichkeit der Herstellung sehr dünner Schichten (<< 1 µm) können sehr kurze Sensoransprechzeiten realisiert werden.
- Die Miniaturisierung und Integration der Erkennungssysteme in mikroelektronische Schaltungen, beispielsweise zur Herstellung von ISFET's und ENFET's, ist problemlos.
- Die selektiven Erkennungssysteme sind grundsätzlich für alle Sensor-Meßanordnungen geeignet.

Anhand von Ausführungsbeispielen soll die Erfindung noch näher erläutert werden.

### Beispiel 1

### Herstellung von Polyether/Enzym-Schichten

100 Masseteile eines epoxyfunktionellen Polyethers der Struktur 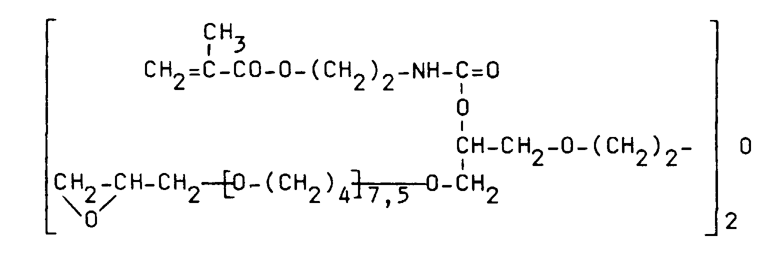 werden mit 7 Masseteilen propoxyliertem Glycerintriacrylat als Reaktivverdünner und 2 Masseteilen 2-Hydroxy-2-methyl-1-phenylpropan-1-on als Photoinitiator gemischt und zur Einstellung der benötigten Verarbeitungseigenschaften mit der entsprechenden Menge Toluol versetzt. Diese Lösung wird dann durch Tauchen, Tropfen oder Rakeln auf die gegebenenfalls mit einem Haftvermittler vorbehandelte sensitive Oberfläche eines Sensors aufgebracht. Parallel dazu werden mit der gleichen Lösung Siliciumwafer mit Hilfe einer Lackschleuder beschichtet; die Schleuderzeit beträgt ca. 10 s.

Die Schichten werden in einer Laminarbox getrocknet und danach unter Stickstoff durch UV-Bestrahlung (Anlage F 450 der Fa. Fusion UV-Curing Systems) im Wellenlängenbereich 200 bis 450 nm vernetzt; Bestrahlungsdauer: 4,6 s. Zur Entfernung löslicher Anteile werden die vernetzten Schichten bei Raumtemperatur 24 h mit Dioxan extrahiert. Zur Erhöhung der Hydrophilie der Schichten wird ein Teil der Epoxidgruppen mit NH-Gruppen-haltigen Verbindungen in Form von Aminosäuren umgesetzt. Dabei hat sich insbesondere eine Lagerung der Schichten in einer 2 %igen Lösung von Prolin oder Glutaminsäure in einer 2:1-Mischung aus Dioxan und Wasser bei 40 bis 60°C bewährt. Anhand parallel behandelter Siliciumwafer kann der Umsatz IR-spektroskopisch verfolgt werden. Ein Umsatz von 50 % ist in den meisten Fällen ausreichend; bei Bedarf können aber auch höhere Werte eingestellt werden.

Die Immobilisierung der Enzyme erfolgt durch Inkubation der Schichten in einer ca. 1 bis 2 %igen Lösung des Enzyms in Wasser bei 20 bis 30°C. Zur Beschleunigung dieses Vorgangs kann die Lösung, je nach Empfindlichkeit des Enzyms, mit 10 bis 50 % Dioxan versetzt werden. Die Immobilisierung ist nach 1 bis 8 h beendet. Verbliebene Epoxidgruppen können durch schonende Umsetzung mit Aminosäuren beseitigt werden. Zum Abschluß werden die Schichten durch intensives Waschen mit Wasser von extrahierbaren Anteilen befreit.

Tabelle 1 enthält eine Zusammenstellung der erfindungsgemäß in 10 µm dicken, identisch vorbehandelten Schichten auf Siliciumwafern bei 30°C innerhalb von 8 h immobilisierten Enzyme sowie die Enzymaktivität bei 25°C.

### Beispiel 2

### Beurteilung der Funktionsstabilität der immobilisierten Enzyme

Zur Beurteilung der Funktionsstabilität erfindungsgemäß immobilisierter Enzyme (Dauer: 8 h) wurden die Aktivitäten der gemäß Beispiel 1 auf Siliciumwafern hergestellten 10 µm dicken Schichten bei 25°C über mehrere Wochen hinweg gemessen (siehe dazu Tabelle 1). Die Aktivität der Glucoseoxidase

**Tabelle 1**

| Enzym | Aktivität | Bestimmungsmethode |
|---|---|---|
| Glucoseoxidase aus Aspergillus niger, lyophil. 240 U/mg | 0,8 U/cm² | Gluc-DH-Methode der Fa. Merck |
| | | |
| Katalase aus Rinderleber, Suspension 65 000 U/mg | 350 U/cm² | Siehe: B.Stellmach, "Bestimmungsmethoden Enzyme", Steinkopff-Verlag, Darmstadt 1988, Seiten 152 bis 155 |
| | | |
| Urease aus Schwertbohnen, lyophil. 100 U/mg | 0,7 U/cm² | Siehe: B. Stellmach, "Bestimmungsmethoden Enzyme", Steinkopff-Verlag, Darmstadt 1988, Seiten 269 bis 271 |
| | | |
| Alkoholdehydrogenase aus Hefe, lyophil. 400 U/mg | 2,0 U/cm² | Siehe: B. Stellmach, "Bestimmungsmethoden Enzyme", Steinkopff-Verlag, Darmstadt 1988, Seiten 11 und 12 |
| | | |
| L-Asparaginase, 50 %ige Lösung in Glycerin 80 U/mg Lösung | 0,6 U/cm² | Siehe: B. Stellmach, "Bestimmungsmethoden Enzyme", Steinkopff-Verlag, Darmstadt 1988, Seiten 63 bis 68 |

wurde über 70 Tage verfolgt, ohne daß eine Abnahme des anfänglichen Wertes festgestellt werden konnte. Parallel dazu wurde nach der in Tabelle 1 angegebenen Bestimmungsmethode die Aktivitätsabnahme einer wäßrigen Glucoseoxidaselösung bei 20°C bestimmt. Dabei ergibt sich ein Aktivitätsverlust von ca. 50 % innerhalb von 10 Tagen, womit die erhöhte Stabilität der erfindungsgemäß immobilisierten Glucoseoxidase belegt wird. Die Beurteilung der anderen in Tabelle 1 aufgeführten immobilisierten Enzyme ergibt den Erhalt des anfänglich gemessenen Aktivitätswertes über mindestens 8 Wochen.

### Beispiel 3

### Beurteilung der Funktionsstabilität von Biosensoren mit immobilisierten Enzymen nach der Erfindung

Nach dem im Beispiel 1 beschriebenen Verfahren werden Polyether/Enzym-Schichten auf Sensor-Meßanordnungen hergestellt und deren Funktion und Funktionsstabilität durch Messung des entstehenden Sensorsignals verfolgt. Tabelle 2 enthält die beurteilten Enzyme sowie die für die Beurteilung gewählte Meßanordnung und die Funktionsdauer.

**Tabelle 2**

| Enzym | Sensor-Meßanordnung | Funktionsdauer |
|---|---|---|
| Glucoseoxidase (GOD) | Sauerstoffsensor nach EP-OS 0 470 473 | > 8 Wochen |
| | | |
| GOD + Katalase (1:1) | Sauerstoffsensor nach EP-OS 0 470 473 | > 8 Wochen |
| | | |
| Urease | NH₄⁺-sensitive Glaselektrode (Fa. Tecan AG) | > 8 Wochen |
| | | |
| L-Asparaginase | NH₄⁺-sensitive Glaselektrode (Fa. Tecan AG) | > 8 Wochen |

## Patentansprüche

1. Biosensor mit einem selektiven Erkennungssystem aus einem Polymer und einer biochemischen Substanz, insbesondere ein Enzym, **gekennzeichnet durch** ein in folgender Weise erzeugbares Erkennungssystem:
- ein olefinisch ungesättigter, epoxyfunktioneller Polyether wird in Form einer Schicht auf ein Trägermaterial aufgebracht,
- der Polyether wird durch energiereiche Strahlung zu einer weitmaschigen epoxyfunktionellen Polymermatrix vernetzt,
- die Schicht wird mit einer wäßrigen Lösung der biochemischen Substanz behandelt, wobei die biochemische Substanz durch Reaktion mit Epoxidgruppen in der Polymermatrix immobilisiert wird,
- die Schicht wird durch Reaktion von nicht umgesetzten Epoxidgruppen mit einer amino- und/oder carboxylgruppenhaltigen Verbindung stabilisiert.

2. Biosensor nach Anspruch 1, **dadurch gekennzeichnet,** daß die Schicht strukturiert ist.

3. Biosensor nach Anspruch 1 oder 2, **dadurch gekennzeichnet**, daß der vernetzte Polyether hydrophiliert ist.

4. Biosensor nach einem der Ansprüche 1 bis 3, **dadurch gekennzeichnet**, daß der Polyether folgende Struktur besitzt: 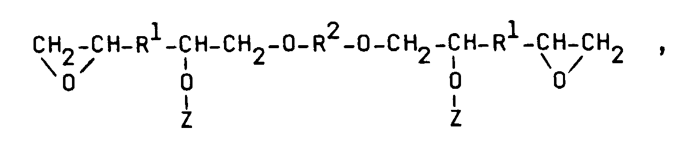 wobei folgendes gilt:
Z = 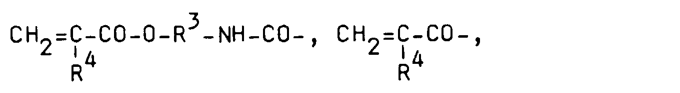 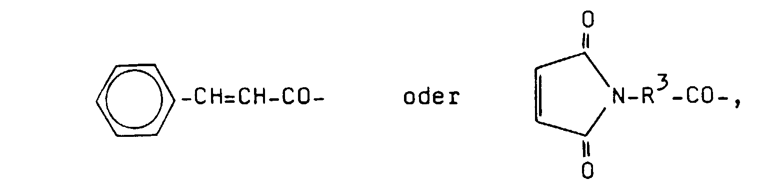 wobei
R³ = -(CH₂)ₘ-, mit m = 1 bis 10,
R⁴ = H oder CH₃;
R¹ = -(CH₂)ₒ-, mit o = 0 bis 18,
-CH₂-O-R⁵-O-CH₂-, wobei
R⁵ = -(CH₂)ₚ-, 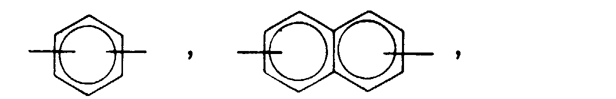 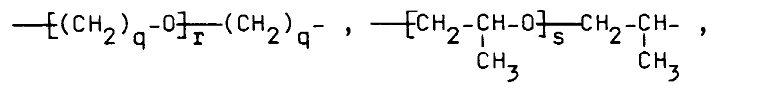 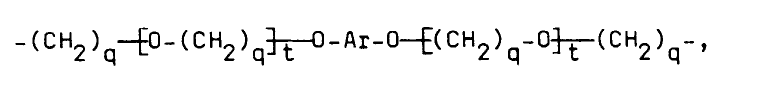 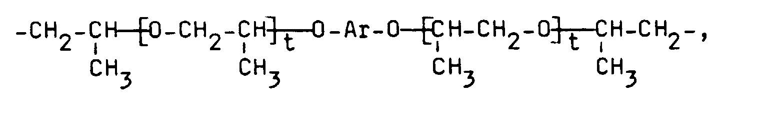
mit
p = 2 bis 20, q = 2 bis 4, r = 1 bis 50,
s = 0 bis 50, t = 0 bis 25, 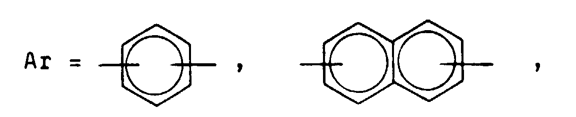 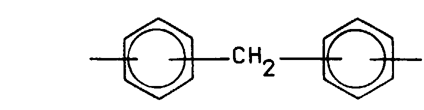 oder 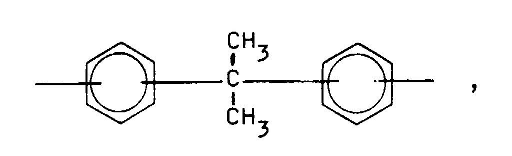 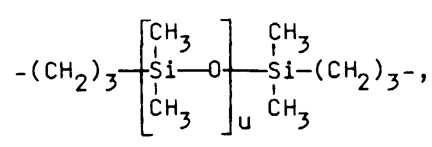 mit u = 0 bis 150,
oder die entsprechende Gruppierung aus 3,4-Epoxycyclohexylmethyl-3',4'-epoxycyclohexancarboxylat;
R² = -(CH₂-CH=CH-CH₂)ₙ- , -R⁶- , -R⁶-O-CO-R⁷-CO-O-R⁶- oder 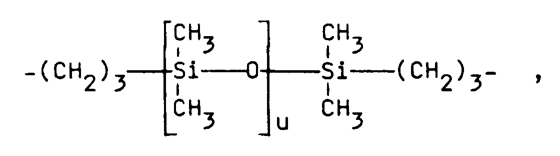 wobei
n = 1 bis 50, u = 0 bis 150,
R⁶ dieselbe Bedeutung hat wie R⁵, außer 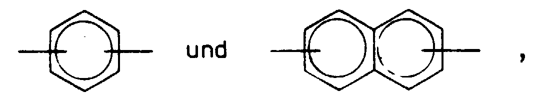 und
R⁷ folgendes bedeutet: 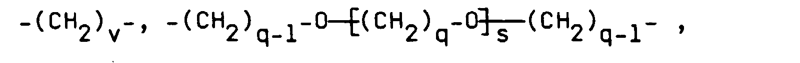 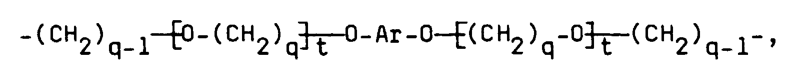
mit
q = 2 bis 4, s = 0 bis 50, t = 0 bis 25,
v = 0 bis 20, und
Ar die vorstehend angegebene Bedeutung hat.

## Claims

1. Biosensor having a selective recognition system comprising a polymer and a biochemical substance, in particular an enzyme, characterised by a recognition system which can be produced in the following way:
- an olefinically unsaturated, epoxy-functional polyether is deposited in the form of a layer onto a carrier material;
- the polyether is cross-linked by high-energy radiation to form a wide-meshed epoxy-functional polymer matrix;
- the layer is treated with an aqueous solution of the biochemical substance, with the biochemical substance being immobilised in the polymer matrix by reaction with epoxide groups; and
- the layer is stabilised by reaction of non-converted epoxide groups with a compound containing amino groups and/or carboxyl groups.

2. Biosensor according to claim 1, characterised in that the layer is structured.

3. Biosensor according to claim 1 or 2, characterised in that the cross-linked polyether is hydrophilized.

4. Biosensor according to one of the claims 1 to 3, characterised in that the polyether has the following structure: 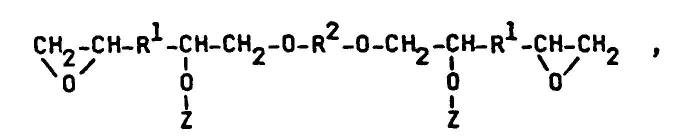 where the following applies:
Z = 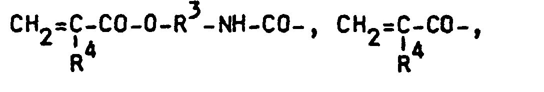 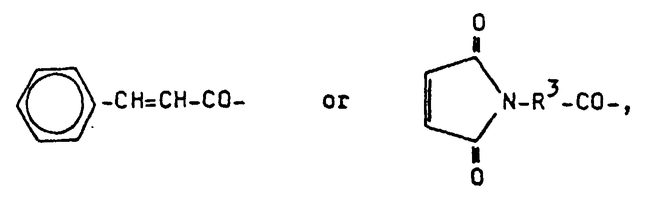 where
R³ = -(CH₂)ₘ-, with m = 1 to 10,
R⁴ = H or CH₃;
R¹ = -(CH₂)_{O}-, with o = 0 to 18,
-CH₂-O-R⁵-O-CH₂-,
where
R⁵ = -(CH₂)ₚ-, 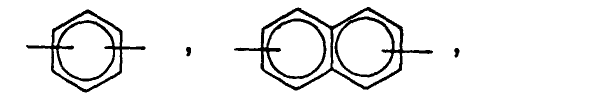 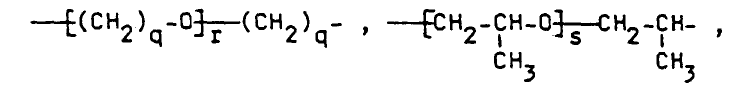 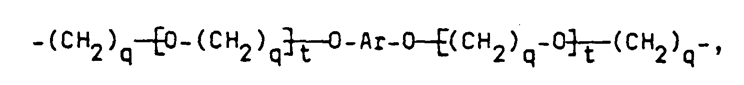 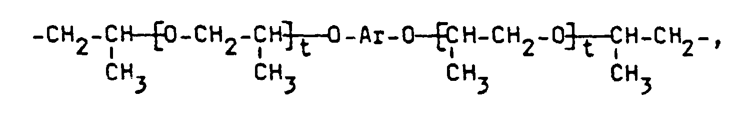 with
p = 2 to 20, q = 2 to 4, r = 1 to 50,
s = 0 to 50, t = 0 to 25, 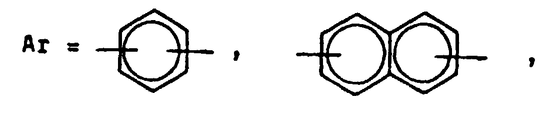 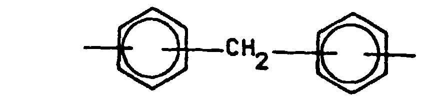 or 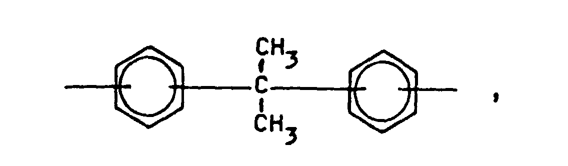 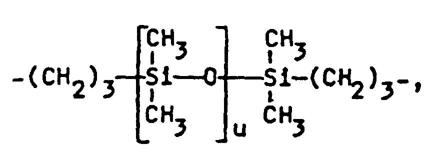 with u = 0 to 150,
or the corresponding grouping of 3,4-epoxycyclohexylmethyl-3',4'-epoxycyclohexane carboxylate;
R² = -(CH₂-CH=CH-CH₂)ₙ-, -R⁶-, -R⁶-O-CO-R⁷-CO-O-R⁶- or 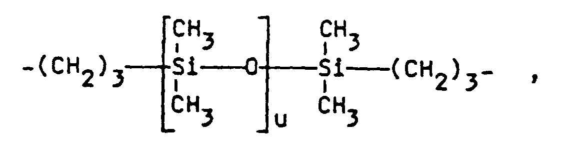 where
n = 1 to 50, u = 0 to 150
R⁶ has the same meaning as R⁵, except 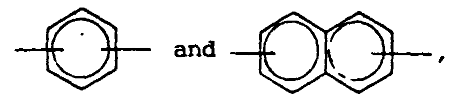 and
R⁷ means the following: 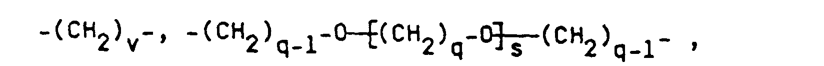 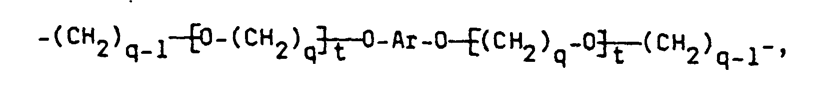
with
q = 2 to 4, s = 0 to 50, t = 0 to 25
v = 0 to 20, and
Ar has the meaning given above.

## Revendications

1. Biocapteur avec un système d'identification sélectif, constitué d'un polymère et d'une substance biochimique, en particulier une enzyme, caractérisé par un système d'identification pouvant être produit de la manière suivante :
- un polyéther oléfiniquement insaturé, à fonction époxy, est disposé sous forme d'une couche, sur un matériau support ;
- le polyéther est réticulé par un rayonnement riche en énergie en une matrice polymère à fonction époxy et à grandes mailles ;
- la couche est traitée avec une solution aqueuse de la substance biochimique, où la substance biochimique est immobilisée dans la matrice polymère par réaction avec des groupes époxyde;
- la couche est stabilisée par réaction des groupes époxyde n'ayant pas réagi avec un composé contenant des groupes amino et/ou carboxyle.

2. Biocapteur selon la revendication 1, caractérisé en ce que la couche est structurée.

3. Biocapteur selon la revendication 1 ou 2, caractérisé en ce que le polyéther réticulé est rendu hydrophile.

4. Biocapteur selon l'une quelconque des revendications 1 à 3, caractérisé en ce que le polyéther possède la structure suivante : 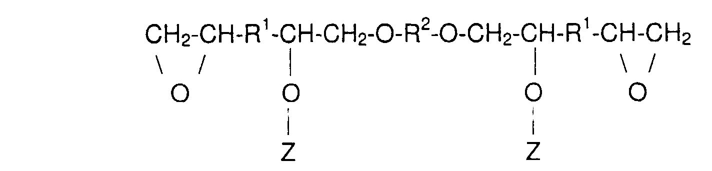
où Z = 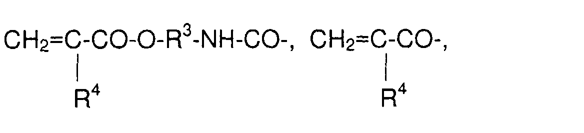 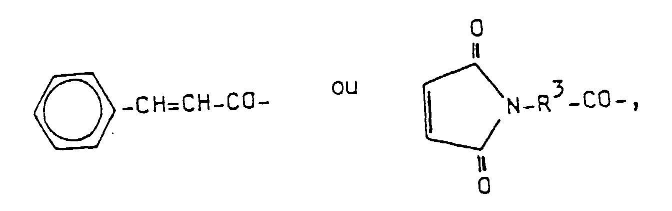
où R³ = -(CH₂)ₘ-, avec m = 1 à 10,
R⁴ = H ou CH₃;
R¹ = -(CH₂)ₒ-, avec o = 0 à 18,
-CH₂-O-R⁵-O-CH₂-,
où R⁵ = -(CH₂)ₚ-, 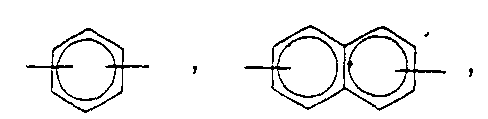 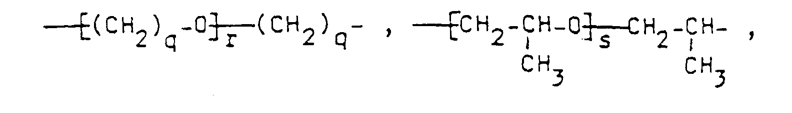 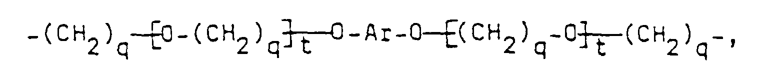 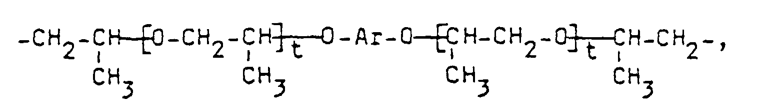
avec p = 2 à 20, q = 2 à 4, r = 1 à 50, s = 0 à 50, t = 0 à 25,
Ar = 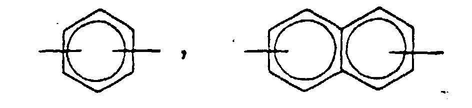 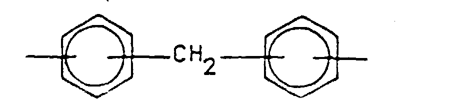 ou 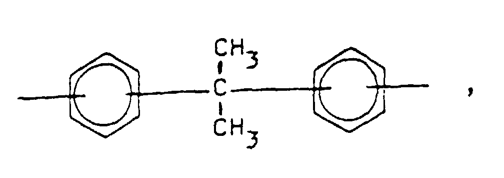 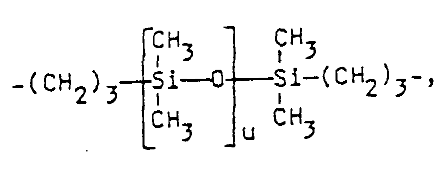
avec u = 0 à 150,
ou le groupement approprié du 3',4'-époxycyclohexanecarboxylate de 3,4-époxycyclohexylméthyle ;
R² = -(CH₂-CH=CH-CH₂)ₙ-, -R⁶-, -R⁶-O-CO-R⁷-CO-O-R⁶- ou 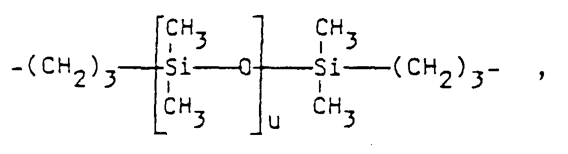
où n = 1 à 50, u = 0 à 150,
R⁶ a la même signification que R⁵,
sauf 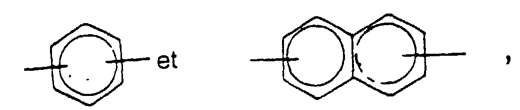 et
R⁷ signifie ce qui suit :
-(CH₂)ᵥ-, -(CH₂)_{q-1}-O[(-CH₂)_{q}-O]ₛ-(CH₂)_{q-1}-,
-(CH₂)_{q-1}[-O-(CH₂)_{q}]ₜ-O-Ar-O[(CH₂)_{q}-O]ₜ-(CH₂)_{q-1}-,
avec q = 2 à 4, s = 0 à 50, t = 0 à 25, v = 0 à 20, et
Ar a la signification donnée précédemment.
